# EUROPÄISCHE PATENTANMELDUNG

(11) **EP 2 168 480 A2**
(43) Veröffentlichungstag der Anmeldung: **31.03.2010**
(21) Anmeldenummer: 09171573.0
(22) Anmeldetag: 29.09.2009
(51) Int. Cl.: A61B 5/083

(54) **Nichtverbrauchendes Gas-Konversionsmodul**

(30) Priorität: 30.09.2008 DE 102008049768
(71) Anmelder: SIEMENS AKTIENGESELLSCHAFT, 80333 München (DE)
(72) Erfinder: Abraham-Fuchs, Klaus, 91058, Erlangen (DE); Hiltawsky, Karsten, 58239, Schwerte (DE); Hornung, Oliver, 90768, Fürth (DE); Krüger-Sundhaus, Thomas, 96178, Pommersfelden (DE); Magori, Erhard, 85622, Feldkirchen (DE); Paulicka, Peter, 91056, Erlangen (DE); Pohle, Roland, 85570, Herdweg (DE); von Sicard, Oliver, 80469, München (DE)

(57) **Zusammenfassung**

Die Anmeldung betrifft eine Vorrichtung (1) zur Messung von Stickstoffmonoxid in Ausatemluft durch eine Gassensoreinheit (13) mit mindestens einem Gassensor (15), wobei eine Einrichtung zur Oxidation (17) von Stickstoffmonoxid zu Stickstoffdioxid vorhanden ist, wobei die Einrichtung zur Oxidation von Stickstoffmonoxid zu Stickstoffdioxid einen nicht verbrauchenden Katalysator zur Katalyse der Oxidation von Stickstoffmonoxid zu Stickstoffdioxid aufweist.

## Beschreibung

Die vorliegende Erfindung betrifft eine Anordnung zur Messung der Konzentration von Stickoxid (NO) im Atemgas und ein Verfahren zur Messung der Konzentration von NO.

Stickoxid (Stickstoffmonoxid, NO) ist ein Marker der aus Zellen der Atemwege kontinuierlich in den Atemgasstrom freigesetzt wird und welcher ein wichtiger Marker bei der Diagnose von Asthma und der Optimierung der Behandlung von Asthma darstellt. Asthma gehört bei ca. 5% der Erwachsenen und ca. 20% der Kinder in entwickelten Industrienationen zu den am häufigsten auftretenden Krankheiten. Bei Entzündungsvorgängen der Atemwege, z.B. Asthma treten erhöhte NO-Konzentrationen von 40 ppb (parts per billion) oder mehr in der Ausatemluft auf. Bevorstehende Asthmaanfälle sind durch einen Anstieg des NO-Gehaltes der Ausatemluft bereits deutlich früher erkennbar als durch einen Lungenfunktionstest. Somit ist die NO-Messung in der Ausatemluft ein bevorzugtes Verfahren zur Diagnose und Therapieverlaufskontrolle von Asthma und anderen entzündlichen Atemwegserkrankungen.

Preiswerte NO-Sensoren mit der erforderlichen Empfindlichkeit im ppb-Bereich waren bisher nicht am Markt verfügbar. Ein neu entwickelter NO₂-Sensor auf Basis der "Suspended Gate FET"-Technologie entspricht den genannten Anforderungen. Allerdings muss einem derartigen Sensor ein Konversionsmodul zur Umwandlung des NO im Atemgas zu NO₂, welches vom Sensor detektiert werden kann, vorgeschaltet werden. Ein derartiges Konversionsmodul sollte idealerweise mehrere Monate oder gar Jahre halten, preiswert sein und NO mit höchstmöglicher und konstanter Umwandlungsrate in NO₂ konvertieren.

Bisher wurden für diesen Zweck Oxidationsmittel verwendet, welche beispielsweise in einer vorgeschalteten Kammer vorgesehen waren, durch die das Atemgas hindurch geleitet wird.

Als Oxidationsmittel sind beispielsweise in der DE 101 212 62 A1 Permanganatsalze und Perchloratsalze beschrieben. Nachteilig bei dieser Lösung ist, dass das Oxidationsmittel selbst verbraucht wird und das Konversionsmodul damit seine Fähigkeit zur Umwandlung von NO in NO₂ mit der Zeit verliert.

Dem gegenüber hat die vorliegende Erfindung die Aufgabe, eine Anordnung zur Messung von NO in der Atemluft bereit zu stellen, welche preiswert und wieder verwendbar ist.

Erfindungsgemäß wird vorgeschlagen, ein Konversionsmodul zu verwenden, welches sich nicht verbraucht bzw. kontinuierlich regeneriert werden kann. Dies wird erreicht durch Einsatz eines nicht verbrauchenden Oxidationskatalysators.

Die Erfindung betrifft insbesondere eine Vorrichtung zur Messung von Stickstoffmonoxid in einer Ausatemluft durch eine Gassensoreinheit mit mindestens einem Gassensor, wobei eine Einrichtung zur Oxidation von Stickstoffmonoxid zu Stickstoffdioxid vorhanden ist, wobei die Einrichtung zur Oxidation von Stickstoffmonoxid zu Stickstoffdioxid einen nicht verbrauchenden Katalysator zur Katalyse der Oxidation von Stickstoffmonoxid zu Stickstoffdioxid aufweist

Gemäß einer ersten Ausführungsform der Erfindung ist der Katalysator ein thermisch aktivierbarer Katalysator. Gemäß einer bevorzugten Ausführungsform dieser ersten Ausführungsform weist die erfindungsgemäße Vorrichtung ferner eine Heizvorrichtung auf.

Gemäß einer alternativen Ausführungsform ist der Oxidationskatalysator ein fotochemischer Katalysator (Fotokatalysator). Gemäß einer bevorzugten Ausführungsform alternativen Ausführungsform weist die erfindungsgemäße Vorrichtung ferner eine Lichtquelle auf.

Gemäß einer weiteren Ausführungsform weist die erfindungsgemäße Vorrichtung ferner eine Einrichtung zur Erzeugung von aktivierten Sauerstoffspezies auf, insbesondere eine Einrichtung zur Erzeugung von Ozon oder Singulett-Sauerstoff.

Gemäß einer bevorzugten Ausführungsform ist der Katalysator bei einer Temperatur von weniger als 300°C, vorzugsweise weniger als 250°C, besonders bevorzugt weniger als 200°C wirksam. Dabei soll der Katalysator bei einer Temperatur von weniger als 300°C, vorzugsweise weniger als 250°C, besonders bevorzugt weniger als 200°C mindestens 80% der Reaktionsrate erreichen, die er in seinem Temperaturoptimum erreicht.

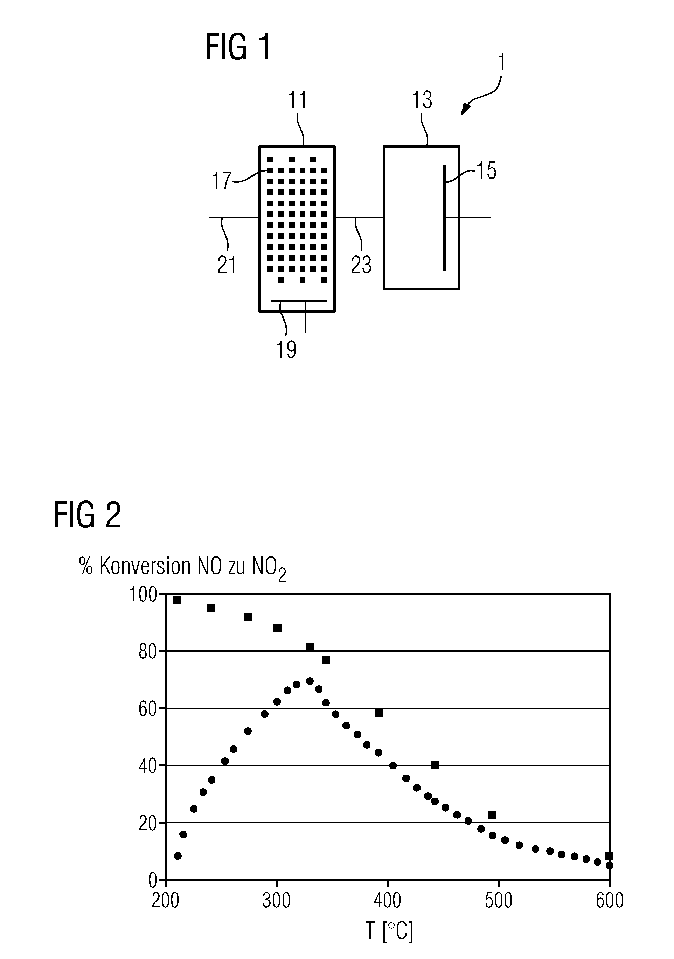

Dies ist besonders vorteilhaft, da bei niedrigeren Temperaturen eine vollständige bzw. vollständigere Konversion von NO zu NO2 möglich ist (siehe dazu auch Fig. 2).

Vorzugsweise wird die Einrichtung zur Oxidation von Stickstoffmonoxid zu Stickstoffdioxid möglichst nahe am Sensor, etwa an der Einlassöffnung zur Messkammer oder in der Messkammer selbst, integriert, damit das konvertierte Gas möglichst unmittelbar gemessen werden kann.

In einer anderen Ausführungsform wird die Einrichtung zur Oxidation von Stickstoffmonoxid zu Stickstoffdioxid im Sensorelement selbst integriert. Dies kann durch einen schichtartigen Aufbau (z.B. Katalysatorschicht auf Sensorschicht) oder einen monolithischen Aufbau (z.B. Katalysator in Sensorschicht dispergiert) erreicht werden.

In einer anderen Ausführungsform der Erfindung wird das Gasanalyse-Gerät (bevorzugt in wählbaren Zeitintervallen) zur Qualitätskontrolle oder Kalibrierung mit einem Kalibriergas von definierter NO-Konzentration beaufschlagt. Dieser Kalibriervorgang kann auch dazu dienen, die Wirkungsrate des Konversionsmoduls zu messen, und bei abfallender Wirkungsrate die Regenerierung zu aktivieren.

Gemäß einer Ausführungsform der Erfindung weist die Gassensoreinheit einen NO₂-sensitiven Feldeffektortransystor-Sensor (FET-Sensor) auf.

Die Umsetzung von Stickstoffmonoxid zu Stickstoffdioxid erfolgt gemäß der folgenden Reaktionsgleichung:

2 NO + O₂ ↔ 2 NO₂, ΔH = -114 kJ/mol

Da die Reaktionsenthalpie negativ ist, erfolgt die Reaktion in Richtung der Konversion zu NO₂, sie muss also nur durch einen Katalysator ermöglich werden. Dazu wird angemerkt, dass auch in ausgeatmeter Luft noch der Großteil des in der Umgebungsluft vorhandenen Sauerstoffs präsent ist, da nur ein kleiner Teil davon beim Atmen verbraucht wird.

In der menschlichen Ausatemluft können sich auch weitere reduzierend wirkende metabolische Beiprodukte (z.B. Ketone oder Alkohole) befinden. Wenn ein Sensorelement versendet wird, welches gegenüber diesem nicht selektiv ist, besteht die Gefahr, dass die Messung gestört wird. Ein weiterer Vorteil der vorliegenden Erfindung liegt darin, dass das bei Verwendung eines wirksamen Oxidationskatalysators diese Beiprodukte zu nicht detektiertem CO₂ und H₂O aufoxidiert werden und die Selektivität der Stickoxidmessung somit erhöht wird.

Die Erfindung wird im Folgenden anhand von Beispielen und im Zusammenhang mit den Figuren beschrieben, in welchen zeigen:
- FIG 1: eine schematische Darstellung einer Ausführungsform der erfindungsgemäßen Vorrichtung;
- FIG 2: eine grafische Darstellung der Konversionsrate von NO zu NO₂ in Abhängigkeit der Temperatur

FIG 1 zeigt beispielhaft und schematisch eine Ausführungsform der erfindungsgemäßen Vorrichtung 1 mit einem Konversionsmodul 11 und einer Gassensoreinheit 13. Über eine Zuleitung 21 wird Ausatemluft in das Konversionsmodul 11 geleitet, in welchem ein Oxidationskatalysator 17 vorgesehen ist. Im Falle eines thermisch aktivierbaren Oxidationskatalysators kann zusätzlich eine Heizvorrichtung 19 vorgesehen sein, z.B. in Form einer elektrisch resistiven Heizvorrichtung. Über eine Leitung 23 wird nach der katalytischen Konversion die Ausatemluft in die Gassensoreinheit 13 geleitet, in welcher ein NO₂-sensitiver Gassensor 15 vorgesehen ist.

Als Gassensor 15 kommt z.B. die Verwendung eines NO2-sensitiven Sensors auf Basis eines Transistors in betracht. Bei Einsatz der Stickoxiddetektion nach dem Prinzip der Austrittsarbeitsmessung sind verschiedene Feldeffekttransistoren bekannt, bei denen die gassensitive Schicht als Gate-Elektrode dargestellt ist. Diese Gate-Elektrode kann durch einen Luftspalt abgetrennt sein von dem sogenannten Kanalbereich des Feldeffekttransistors. Grundlage für ein detektierendes Messsignal ist die Änderung des Potentials zwischen Gate und Kanalbereich (ΔV_{G}). In den deutschen Patentanmeldungen Nr. 198 14 857.7 und Nr. 199 56 806.5 werden beispielsweise hybride Flip-Chip-Aufbauten von Gassensoren beschrieben, die als CMOS-Transistoren ausgeführt sind. Ein Gassensor kann darüber hinaus mit zwei Feldeffekttransistoren bestückt sein, deren Regelverhalten durch annähernd gleichgroße Luftspalte zwischen Kanalbereich und Gateelektrode angeglichen ist und deren Sensorschichten separat auslesbar sind. In der deutschen Patentanmeldung Nr. 199 56 744.1 wird beschrieben wie die Beabstandung zwischen Gate-Elektrode und Kanalbereich eines Feldeffekttransistors durch äußerst präzise Abstandshalter reproduzierbar darstellbar ist. Eine andere Ausgestaltung sieht vor, das gassensitive Material in poröser Form auf dem Kanalbereich oder dem Gate aufzubringen.

Gassensitive Schichten zum Einsatz in einem sogenannten SG-FET (Suspended Gate-Feldeffekttransistor) können vorteilhaft Porphinfarbstoffe sein, wie z.B. Phthalocyanine mit dem Zentralatom Kupfer oder Blei. Bei Sensortemperaturen zwischen 50° und 120° C können Stickoxidsensitivitäten bis in den unteren ppb-Bereich nachgewiesen werden. Die Detektion zielt wie üblich auf Stickstoffdioxid ab.

Andere zum Einsatz in gassensitiven Feldeffekttransistoren geeignete Materialien als gassensitive Schichten zur Detektion von Stickoxid, insbesondere von Stickstoffdioxid, sind bei Temperaturen zwischen 80° und 150° C betriebene feinkristalline Metalloxide. Dies können insbesondere SnO₂, WO₃, In₂O₃ sein, Salze aus dem Karbonatsystem wie Bariumkarbonat oder Polymere wie beispielsweise Polysiloxane, sind ebenfalls denkbar.

Katalysatoren, die auf einer sehr fein verteilten Dispersion eines Edelmetallkatalysators, z.B. Platin, Rhodium oder Palladium, basieren, sind geeignet die Konversion mit bester Effizienz mit niedriger Temperatur durchzuführen. Dazu gehören z.B. Platinmohr (Carbonplatin), ein sogenannter "getragener Katalysator", also eine Katalysatordispersion, die auf einem Trägerkörper aufgebracht ist. Als Trägerkörper geeignet sind offenporige von Gas durchströmbare Metalloxid- oder Keramikträgerkörper, extrudierte, nicht poröse Oxid-Formkörper mit integrierter Kanalstruktur, in welchem das Gas durch die Kanäle strömt, um maximalen Kontakt mit der Katalysatoroberfläche sicherzustellen, und ähnliche. Alternativ kann auch ein ausreichend thermisch stabiles Polymer als Träger für den Katalysator vorgesehen werden. Geeignete Materialien sind z.B. PMMA, PDMA oder Polyimide. Hierzu wird ein Formkörper mit Mikrotechnologieverfahren erzeugt, der mit Mikrokanälen durch Strömung versehen ist.

Es kann auch ein feines Netz oder ein faserartiger Aufbau aus kleinen katalytisch aktiven Drähten oder Fasern (z.B. Platin) vorgesehen werden. Da derartige Aufbauten auch elektrisch leitfähig sind, können sie vorteilhaft gleichzeitig zur Beheizung genutzt werden.

Für eine hohe Oxidationswirkung gegenüber NO können beispielsweise Oxide von Seltenen Erdmetallen und/oder von Übergangsmetallen als Katalysatormaterial, als Katalysatorbestandteil bzw. Beschichtungsbestandteil vorgesehen sein. Vorteilhaft ist insbesondere der Einsatz eines oder mehrerer Oxide aus der Gruppe V2O5, Cr2O3, Mn2O3, MnO2, Mn3O4, Fe2O3, Fe3O4, CoO, CO3O4, NiO, NiO2, Ni2O3, CeO2, Ce2O3. Diese Katalysatoren können auch als Zusatz in Edelmetallkatalysatoren vorgesehen sein.

Oxide von Seltenen Erdmetallen und/oder von Übergangsmetallen als Katalysatormaterial oder Katalysatorzusatz haben den Vorteil, dass diese Katalysatoren bei niedrigeren Temperaturen unterhalb 250° wirksam sind. Dieser Vorteil ist auch bei Fotokatalysatoren gegeben.

In Figur 2 ist grafisch die Konversion von Stickstoffmonoxid zu Stickstoffdioxid in Abhängigkeit der Temperatur dargestellt. Bei Temperaturen von >200°C findet eine fast vollständige Konversion zu NO₂ statt. Bei steigenden Temperaturen verschiebt sich das Gleichgewicht in Richtung NO. Dies ist in der Figur durch Quadrate dargestellt. In der Grafik überlagert ist schematisch und dimensionslos die Konversionsrate (Kreis-Symbole) durch den Katalysator (in diesem Beispiel: Pt) in Abhängigkeit der Temperatur dargestellt. Bei niedrigen Temperaturen ist zwar das chemische Gleichgewicht stark auf Seite der Konversion zu NO₂, jedoch ist die Reaktionsgeschwindigkeit zu langsam. Durch Einsatz eines geeigneten Katalysators kann die Konversion bei Temperaturen von 150°C bis 250°C stattfinden, wobei in diesem Bereich eine vollständige oder fast vollständige Konversion von NO zu NO₂ stattfinden kann.

Gemäß einer weiteren Ausführungsform wird der Katalysator kurz vor der Umsetzung auf eine höhere Temperatur beheizt (300-450°C), wobei sich reaktive Sauerstoffspezies auf der Oberfläche sammeln. Dann wird der Katalysator abgekühlt und die Umsetzung durchgeführt, wobei durch die tiefere Temperatur wieder eine vollständige Umsetzung zu NO₂ gewährleistet wird. Bei einer derartigen Ausführungsform ist eine entsprechende Steuervorrichtung vorgesehen, welche kurz vor der Messung den Katalysator erhitzt. Vorzugsweise weist diese Steuervorrichtung eine Signalvorrichtung auf, welche nach dem Erhitzen des Katalysators und dem anschließenden Abkühlen auf die Konversionstemperatur signalisiert, dass nun die Messung erfolgen kann. Ferner weist die Steuervorrichtung bevorzugt einen Temperatursensor auf.

Zur Senkung der benötigten Temperaturen kann die katalytische Wirkung durch Zugabe katalytischer Promotoren noch verbessert werden, z.B. Metalle wie Rodium, Renium, Osnium oder Oxide wie CER-, Eisen-, Hafnium- oder Lantanoxid.

Alternativ kann die Oxidation des Stickstoffmonoxids auch mittels eines Fotokatalysators geschehen. Bei dieser Ausführungsform ist keine Heizung erforderlich. Es muss jedoch Lichtenergie zugefügt werden. Dazu ist vorzugsweise eine Lichtquelle vorzusehen. Üblicherweise erfolgt die Zuführung von UV-Licht, z.B. durch eine UV-LED oder eine UV-Lampe. Als Fotokatalysator ist bevorzugt TiO₂ vorzusehen. Vorteilhaft wird TiO₂ in der reaktiven Kristallstruktur anastas verwendet und ferner eine hohe Reaktivität durch eine entsprechend geringe Korngröße des Katalysators (bevorzugt geringer als 50 nm) zu erreichen.

Die Katalysatoren besitzen im Allgemeinen eine limitierte Umsetzungskapazität. Zur Optimierung des Katalysatorvolumens und der benötigten Temperatur wird vorteilhaft ein Aufbau vorgesehen, der die Führung nur eines Teiles des Gasstroms durch den Katalysator vorsieht. Der andere Teil des Gasstroms wird nicht dem Sensor zugeführt und verlässt das Gerät. Dies erlaubt eine längere Verweilzeit im Katalysator und damit eine verbesserte Umsetzung.

Ferner ist zu beachten, dass Katalysatoren an ihrer Oberfläche eine gewisse Speicherfähigkeit für Stickoxide besitzen, welche zu Messverfälschungen führen kann. Daher ist der Aufbau bevorzugt so zu gestalten, dass der Katalysator vor der Messung ausreichend lange (z.B. 2 sek.) mit Atemgas gespült wird, so dass im Folgen kein Stickoxid durch Katalysatorspülungen verloren geht. Nach der Messung wird der Katalysator bevorzugt mindestens für dieselbe Zeitdauer mit stickoxidfreier Luft gespült, um eine Verschleppung von angelagerten Stickoxiden in den nächsten Messzyklus zu vermeiden. Bevorzugt ist eine entsprechende Steuerung vorzusehen, welche die Gasströmung misst, und signalisiert, dass der Katalysator für eine ausreichend lange Zeitdauer vor der Messung mit Atemluft bzw. nach der Messdauer mit stickoxidfreier Luft gespült wurde.

Ferner kann die Umsetzung von NO zu NO₂ durch Generierung aktiver Sauerstoffspezies unterstützt werden. Dies kann insbesondere durch Ozon (O₃) geschehen, welches z.B. durch eine integrierte UV-Lichtquelle oder durch eine miniaturisierte elektrische Entladung (CORONA-Typ) erzeugt wird. Zur vollständigen Reaktion des Stickoxids mit dem so gebildeten Ozon ist jedoch weiterhin eine katalytisch aktive Oberfläche notwendig. Ein weiteres Beispiel für eine aktivierte Sauerstoffspezies ist Singulett-Sauerstoff, eine hochreaktive angeregte Variante des üblicherweise im Triplett-Zustand vorliegenden O₂-Moleküls. Singulett-Sauerstoff reagiert sofort mit anderen Gasen und die Erzeugung von Singulett-Sauerstoff erfolgt fotochemisch durch Lichtbestrahlung geeigneter Farbstoffe, z.B. Methylenblau, Eosin, Bengalrosa oder auch Phthalocyanin, z.B. mit Licht im Nahinfrarotbereich aus einer entsprechenden LED. Üblicherweise wird eine Wellenlänge von 850 - 1250 nm verwendet.

Vorzugsweise wird das Konversionsmodul möglichst nahe am Sensor, z.B. an der Einlassöffnung zur Messkammer oder in der Messkammer selbst integriert vorgesehen, damit das konvertierte Gas möglichst unmittelbar gemessen werden kann.

Gemäß einer weiteren Ausführungsform wird das Konversionsmodul im Sensorelement selbst integriert (hybrid oder monolytisch). Dies kann durch einen Zweischichtaufbau erfolgen (eine Katalysatorschicht auf der Sensorschicht) oder durch einen monolytischen Aufbau (die Sensoroberfläche befindet sich auf dem gleichen Trägerkörper und ist homogen oder heterogen mit dem katalytisch aktiven Material durchmischt). Bevorzugt ist auf der Oberfläche oder im Material des Konversionsmoduls integriert eine Heizvorrichtung, welche die oxidative Fähigkeit des Moduls wieder regeneriert. Die Heizvorrichtung kann automatisch in Betrieb gesetzt werden, gesteuert z.B. durch die Messung der Betriebsstunden oder durch die Messung des Gasflusses durch das Modul. In einer anderen Ausführungsform wird das Gasanalysegerät in wählbaren Zeitintervallen zur Qualitätskontrolle oder Kalibrierung mit einem Kalibriergas von definierter NO-Konzentration beaufschlagt. Dieser Kalibriervorgang kann auch dazu dienen, die Wirkungsrate des Konversionsmoduls zu messen und bei abfallender Wirkungsrate die Regenerierung zu aktivieren.

Wesentliche Vorteile des Gesamtsystems liegen darin, dass eine nicht invasive Messmethode verwendet wird. Die Messungen sind in großer Anzahl wiederholbar und können somit auch zur Verlaufskontrolle bei Therapien, bei der Diagnose von Asthma bei Kindern, bei der Früherkennung von Asthma oder für vorbeugende medizinische Maßnahmen besonders eingesetzt werden. Durch Einsatz nicht verbrauchender Katalysatoren ist die Erfindungsgemäße Vorrichtung wartungsarm und ermöglicht kostengünstige Messungen. Das hier vorgestellte System ist deshalb auch für den Einsatz außerhalb von Kliniken und Arztpraxen geeignet.

## Patentansprüche

1. Vorrichtung zur Messung von Stickstoffmonoxid in Ausatemluft durch eine Gassensoreinheit mit mindestens einem Gassensor, wobei eine Einrichtung zur Oxidation von Stickstoffmonoxid zu Stickstoffdioxid vorhanden ist, **da durchgekennzeichnet,**
**dass** die Einrichtung zur Oxidation von Stickstoffmonoxid zu Stickstoffdioxid einen nicht verbrauchenden Katalysator zur Katalyse der Oxidation von Stickstoffmonoxid zu Stickstoffdioxid aufweist.

2. Vorrichtung nach Anspruch 1, wobei der Katalysator ein thermisch aktivierbarer Katalysator ist.

3. Vorrichtung nach Anspruch 2, ferner aufweisend eine Heizvorrichtung.

4. Vorrichtung nach Anspruch 1, wobei der Katalysator ein fotochemischer Katalysator ist.

5. Vorrichtung nach Anspruch 4, ferner aufweisend eine Lichtquelle.

6. Vorrichtung nach einem der vorangehenden Ansprüche, ferner aufweisend eine Einrichtung zur Erzeugung von aktivierten Sauerstoffspezies.

7. Vorrichtung nach einem der vorangehenden Ansprüche, wobei der Gassensor ein NO₂-sensitiver FET-Sensor ist.

8. Vorrichtung nach einem der vorangehenden Ansprüche , wobei die Einrichtung zur Oxidation von Stickstoffmonoxid zu Stickstoffdioxid an der Einlassöffnung zur Messkammer oder in der Messkammer selbstvorgesehen ist.

9. Vorrichtung nach einem der vorangehenden Ansprüche , wobei die Einrichtung zur Oxidation von Stickstoffmonoxid zu Stickstoffdioxid im Sensorelement selbst integriert vorgesehen ist.

10. Vorrichtung nach einem der vorangehenden Ansprüche, ferner aufweisend eine Einrichtung zum Beaufschlagen der Vorrichtung mit einem Kalibriergas von definierter NO-Konzentration.

11. Vorrichtung nach einem der vorangehenden Ansprüche, wobei der Katalysator bei einer Temperatur von weniger als 300°C, vorzugsweise weniger als 250°C, besonders bevorzugt weniger als 200°C wirksam ist.
